# EUROPEAN PATENT APPLICATION

(11) **EP 1 582 591 A2**
(43) Date of publication of application: **05.10.2005**
(21) Application number: 05090046.3
(22) Date of filing: 25.02.2005
(51) Int. Cl.: C12N 15/11, A61K 31/713, C12N 5/10, A01K 67/027

(54) **Sirna and their use for knock down expression of porcine endogenous retrovirus**

(30) Priority: 27.02.2004 EP 04090075
(71) Applicant: Bundesrepublik Deutschland vertreten durch das Bundesminsterium für Gesundheit, dieses vertr. durch das Robert-Koch-Institut, 13353 Berlin (DE)
(72) Inventor: Karlas, Alexander, 10249 Berlin (DE); Denner, Joachim, Dr., 10625 Berlin (DE); Kurth, Reinhardt, Prof.Dr., 14532 Kleinmachnow (DE)
(74) Representative: Ziebig, Marlene

(57) **Abstract**

Transplantation of porcine xenografts into human recipients is a realistic option to overcome the growing worldwide shortage of suitable allogeneic organs. However, there remains the risk of infection by porcine endogenous retroviruses (PERVs) that cannot be eliminated like that by other microorganisms by breeding pigs under specified pathogen free conditions. To reduce the release of PERVs by porcine transplants a new approach, RNA interference (RNAi), was applied. Here we show significant reduction of PERV expression by synthetic short interfering RNAs (siRNAs) corresponding to different parts of the viral genes *gag, pol* and *env*. The most inhibitory sequences were selected and expressed as short hairpin RNAs (shRNAs) by a polymerase III vector system leading to persistent suppression of PERV replication. Cells or organs from transgenic pigs producing such shRNAs should increase the safety of xenotransplantation.

SEQUENCE LISTING

<110> Bundesrepublik Deutschland, vertreten durch das Bu

<120> siRNA and their use for knock down expression of
      porcine endogenous retrovirus

<130> P260904EP

<140>
<141>

<150> 04090075.5
<151> 2004-02-27

<160> 29

<170> PatentIn Ver. 2.1

<210> 1
<211> 21
<212> DNA
<213> viral

<400> 1
aacattcgat gttggatggc c                                           21

<210> 2
<211> 21
<212> DNA
<213> viral

<400> 2
aatgagattg acatgggatt t                                           21

<210> 3
<211> 21
<212> DNA
<213> viral

<400> 3
aagcctggga ccaatgatta t                                           21

<210> 4
<211> 21

<212> DNA
<213> viral

<400> 4
aaggacgctg acaaattgac t                                                21

<210> 5
<211> 21
<212> DNA
<213> viral

<400> 5
aatgctaatc cttccagaat a                                                21

<210> 6
<211> 21
<212> DNA
<213> viral

<400> 6
aagatattgg ggattgattg g                                                21

<210> 7
<211> 21
<212> DNA
<213> viral

<400> 7
aaagctcagg acaggtagag a                                                21

<210> 8
<211> 21
<212> DNA
<213> viral

<400> 8
aacctagtcc gtagttatgg g                                                21

<210> 9
<211> 21
<212> DNA
<213> viral

<400> 9
aatgtgacca aagagcatag a                                                21

<210> 10
<211> 21
<212> DNA
<213> Artificial Sequence

<220>
<223> Description of Artificial Sequence: negative
      control of siRNA target sequence

<400> 10
aattctccga acgtgtcacg t                                                21

<210> 11
<211> 20
<212> DNA
<213> Artificial Sequence

<220>
<223> Description of Artificial Sequence: primer Gag1 up

<400> 11
tccagggctc ataatttgtc                                                  20

<210> 12
<211> 20
<212> DNA
<213> Artificial Sequence

<220>
<223> Description of Artificial Sequence: primer Gag1
      down

<400> 12
tgatggccat ccaacatcga                                                  20

<210> 13
<211> 22
<212> DNA
<213> Artificial Sequence

<220>

<223> Description of Artificial Sequence: primer Gag2 up

<400> 13
accgctgcgc acctatggcc ct                                                22

<210> 14
<211> 24
<212> DNA
<213> Artificial Sequence

<220>
<223> Description of Artificial Sequence: primerGag2
      down

<400> 14
aaattcttct cttgccgtct atca                                              24

<210> 15
<211> 26
<212> DNA
<213> Artificial Sequence

<220>
<223> Description of Artificial Sequence: primer Pol1 up

<400> 15
gctacaacca ttaggaaaac taaaag                                            26

<210> 16
<211> 24
<212> DNA
<213> Artificial Sequence

<220>
<223> Description of Artificial Sequence: primer Pol1
      down

<400> 16
gagggtcacc tgagggtgtt ggat                                              24

<210> 17
<211> 20
<212> DNA
<213> Artificial Sequence

<220>
<223> Description of Artificial Sequence: primer Pol2 up

<400> 17
accctttatg tggatgagcg                                                    20


<210> 18
<211> 20
<212> DNA
<213> Artificial Sequence

<220>
<223> Description of Artificial Sequence: primer Pol2
      down

<400> 18
cgttggtcat ccatcggtct                                                    20


<210> 19
<211> 20
<212> DNA
<213> Artificial Sequence

<220>
<223> Description of Artificial Sequence: primer
      Pol3-4-5 up

<400> 19
tcaggatggg tagaggctta                                                    20


<210> 20
<211> 19
<212> DNA
<213> Artificial Sequence

<220>
<223> Description of Artificial Sequence: primer
      Pol3-4-5 down

<400> 20
gtaagggtct ctttaatgg                                                     19


<210> 21

<211> 19
<212> DNA
<213> Artificial Sequence

<220>
<223> Description of Artificial Sequence: primer Gp70 up

<400> 21
atgcatccca cgttaagcc                                                    19


<210> 22
<211> 24
<212> DNA
<213> Artificial Sequence

<220>
<223> Description of Artificial Sequence: primer Gp70
      down

<400> 22
ctcttttgg tcggttatac cgat                                               24


<210> 23
<211> 25
<212> DNA
<213> Artificial Sequence

<220>
<223> Description of Artificial Sequence: primer Pol-PK1

<400> 23
ttgacttggg agtgggacgg gtaac                                             25


<210> 24
<211> 24
<212> DNA
<213> Artificial Sequence

<220>
<223> Description of Artificial Sequence: primer Pol-PK6

<400> 24
gagggtcacc tgagggtgtt ggat                                              24

<210> 25
<211> 21
<212> DNA
<213> Artificial Sequence

<220>
<223> Description of Artificial Sequence: primer SD up

<400> 25
tgctgtttgc atcaagaccg c                                                21


<210> 26
<211> 21
<212> DNA
<213> Artificial Sequence

<220>
<223> Description of Artificial Sequence: primer SD down

<400> 26
acagacactc agaacagaga c                                                21


<210> 27
<211> 22
<212> DNA
<213> Artificial Sequence

<220>
<223> Description of Artificial Sequence: primer SA down

<400> 27
atggaggcga agcttaaggg ga                                               22


<210> 28
<211> 64
<212> DNA
<213> Artificial Sequence

<220>
<223> Description of Artificial Sequence: sense
      oligonucleotide for cloning

<400> 28
gatccccgga cgctgacaaa ttgactttca agagaagtca atttgtcagc gtcctttttg 60
gaaa                                                                   64

<210> 29
<211> 64
<212> DNA
<213> Artificial Sequence

<220>
<223> Description of Artificial Sequence: antisense
      oligonucleotide for cloning

<400> 29
agcttttcca aaaaggacgc tgacaaattg acttctcttg aaagtcaatt tgtcagcgtc 60
cggg 64

## Description

The invention relates to siRNA molecules, vectors comprising such siRNA, and the use of such siRNA and vectors for increasing the safety of xenotransplantation.

Xenotransplants from pigs may offer a potential solution to the lack of allotransplants (Cooper, 2003). However this requires prevention of the immunological rejection and a better physiological combatibility of the xenotransplant as well as prevention of transmission of porcine microorganisms. The porcine endogenous retroviruses (PERVs) are particularly problematic since they are integrated into the genome of all pigs (Patience et al., 2001) and can infect human cells *in vitro* (Patience, Takeuchi, and Weiss, 1997; Specke, Rubant, and Denner, 2001). Knock-out animals may prove difficult to produce due to the presence of several replication competent and numerous defective proviruses capable of complementing each other. Although PERVs infect human cells *in vitro,* no virus transmission was observed in initial clinical xenotransplantations carried out to treat diabetes or acute liver failure (Irgang et al., 2003; Paradis et al., 1999). In these trials the porcine cells were encapsulated, the contact time was very short or the immunosuppression of the transplant recipient was very weak. In contrast, the conditions envisaged for future xenotransplantation include long term transplantation of porcine cells or whole organs, direct contact between porcine and human cells and pronounced immunosuppression. In the meantime, several experimental pig to non-human primate xenotransplantations and infection experiments using high doses of virus in small animals and non-human primates under immunosuppression have been performed. In none of these was transmission of PERV observed (Denner et al., 2001; Loss et al., 2001; Specke et al., 2000). Nevertheless, the risk of transmission of porcine viruses to transplant recipients remains and with it the risk of retrovirus-induced tumours and/or immunodeficiencies (Denner, 1998).

Therefore, the technical problem underlying the present invention is to provide a ready and safe source of xenotransplants.

This problem is solved by the provision of the embodiments as defined in the claims.

It has been surprisingly discovered that siRNA of the invention are effective and safe molecules for producing cells, organs or animals as source for xenotransplants.

An important aspect of the invention is the inhibition of the expression of PERVs by siRNAs at the level of RNA, protein and virus release. A further aspect of the invention is the suppression of a retrovirus other than HIV-1 and the suppression of an endogenous retrovirus.

To minimize the possibility of PERV transmission different strategies may be developed such as the selection of low virus producer animals (Oldmixon et al., 2002) or the development of an antiviral vaccine (Fiebig et al., 2003). In addition, new approaches such as the use of RNAi, based on the sequence specific degradation of target mRNA induced by short double-stranded RNA should be followed. The mechanism of RNAi is highly conserved among plants, flies, worms and mammals (Caplen et al., 2001). After processing by the ribonuclease III (RNase III)-like enzyme Dicer, double stranded RNA is cleaved into small double stranded fragments (21-25nt) (Hammond et al., 2000). In the cytoplasm these short interfering RNAs (siRNA) interact with the RNA induced silencing complex (RISC), which recognises the target mRNA, homologous to the sequence of the siRNA. Nucleases, as part of the complex, degrade the target RNA and prevent protein translation (Billy et al., 2001; Yang, Lu, and Erickson, 2000). Transfecting mammalian cells with longer dsRNA (>30nt) was found to be problematic because the induced interferon response led to unspecific degradation of mRNA and finally to apoptosis. The Dicer processing step can be bypassed by transfecting cells with synthetic siRNAs (Elbashir, Lendeckel, and Tuschl, 2001). As these siRNAs within the cells are diluted out during cell division, the time of gene silencing activity is limited to approximately four to eight cell doublings (McManus et al., 2002; Stein et al., 2003). Expressing shRNA, that contains a short loop sequence joining the forward and reverse strand, can overcome this limitation. Defined shRNA without any cap- or polyA-structure can be expressed by polymerase-III-dependent promoters such as the murine or human U6 snRNA- or the human RNase P (H1) RNA-promoters (Brummelkamp, Bernards, and Agami, 2002; Paddison et al., 2002; Paul et al., 2002; Sui et al., 2002). In addition, cells with stable chromosomal integration and permanent inhibition of target genes can be obtained if adequate selection markers are used.

The following examples illustrate the preferred methods for the preparation of the compounds of the present invention, which are not intended to limit the scope of the invention thereto.

### Examples

### Materials and methods

### Synthetic siRNAs

The siRNAs gag1, pol2, pol4 and pol5 were purchased from Dharmacon (Lafayette, Colorado), gag2, poll, pol3, pol4, env1, env2 and the control siRNA (fluorescence labelled, without significant homology to mammalian genes) from Qiagen (Hilden, Germany). Sequence information can be found as Supplementary Table 1 online.

### Plasmids

The plasmid pSuper (Suppression of endogenous RNA) was purchased from Oligoengine (Seattle, Washington). Oligonucleotides were ordered from Sigma-Genosys (Steinheim, Germany): (sense: 5' - GATCCCCGGACGCTGACAAATTGACTTTCAAGAGAAGTCAATTTGTCAGCGTCCTTTTT GGAAA-3' and antisense 5'-AGCTTTTCCAAAAAGGACGCTGACAAATTGACTTCTCTTGAAAGTCAATTTGTCAGCGT CCGGG-3'). Oligonucleotides were annealed and ligated to the plasmid pSuper, previously digested with BglII and HindIII, resulting in pSuper-pol2.

### Cells and transfection

Uninfected and PERV infected 293 human kidney cells were cultured in DMEM containing 10 % FCS, penicillin (100 U/ml) and streptomycin (100 µg/ml).

pSuper-pol2 (12µg) and pHygEGFP (1µg, BD Biosciences, Franklin Lakes, New Jersey) plasmid DNA were transfected with TransFast transfection reagent (Promega, Madison, Wisconsin) into 5x 10⁶ PERV-infected 293 cells according to the manufacture's protocol. PERV-B was kindly provided by David Onion, Q-One Biotech Ltd, University of Glasgow, Glasgow, UK. Forty-eight hours post transfection, hygromycin (200µg/ml) was added, and, after 10 days, individual clones were isolated using cloning discs (Sigma, Munich, Germany).

For transfection of synthetic siRNAs, 7500 cells/well were seeded in a 96-well-plate. The next day medium was replaced by 100 µl fresh DMEM containing 10% FCS. siRNAs were diluted in 25 µl Opti-MEM (Invitrogen) and 1 µl of the transfection reagent GeneEraser (Stratagene, La Jolla, California) was added. After 15 minutes incubation at room temperature, the mixture was added to the cells resulting in a final siRNA concentration of 20 nM. All assays were performed in triplicate.

### Western blot analysis

Cells were lysed with RIPA buffer (150mM NaCl, 10mM Tris, pH 7.2, 0.1% SDS, 1% Triton X-100, 1% deoxycholate, 5mM EDTA, 1mM phenylmethylsulfonyl fluoride, 10mM benzamidine, 2 µg/ml leupeptin, 100µM sodium orthovanadate, 10mM p-nitrophenylphosphate) and proteins were subjected to preparative SDS-PAGE (10 µg cell lysate/lane) and transferred to PVDF-membranes by electroblotting. The level of PERV Gag expression was analysed by Western-Blot techniques using a goat anti-p27Gag antiserum (Irgang et al., 2003). Loading of equal amounts of protein was verified using antibodies against β-actin (Sigma).

### Immunofluorescence

Stably transfected cells (2x10⁵/well) were seeded in a 6-well plate onto glass cover slips. The next day, cells were washed with PBS, fixed with 3% formaldehyde and incubated for 30 minutes with Triton X-100. After treatment with the primary (anti-p27Gag, 1:100)(Irgang et al., 2003) and secondary antibody (anti-goat IgG TRITC conjugate, 1:2000, Sigma) at room temperature for one hour, cells were covered with the ProLong Antifade Kit (Molecular Probes, Eugene, Oregon) and mounted on slides upside down.

### Quantitative real-time RT-PCR

Cellular RNA was extracted from PERV infected cells using the RNAeasy Kit (Qiagen) according to the manufacture's protocol or (for the 96-well format) by lysing cells with 100 µl/well "cells-to-cDNA II cell lysis buffer" (Ambion, Austin, Texas). DNA was removed by adding DNaseI (Hoffmann-La Roche, Basel, Switzerland) (0.04 U/µl final, 37°C, 30 min.), which was inactivated by incubating at 75 °C for 10 minutes. cDNA from 5 µl lysate was synthesised with the RT-Kit (Applied Biosystem, Foster City, California) using random decamers (Ambion). To evaluate the degradation of PERV gag-pol mRNA a SYBR-Green real-time PCR was performed with the real-time PCR cycler MX-4000 (Stratagene) and the Brilliant SYBR Green QPCR Master-Mix (Stratagene). The amount of gag-pol and env was calculated relatively to the level of *GAPDH* RNA using the ΔΔct-method (Livak and Schmittgen, 2001). The amount of PERV mRNA degradation after siRNA treatment was normalised to cells transfected with the control siRNA. For sequence information of all used oligonucleotides please see the Supplementary Table 2 online.

### In vitro infection assay

Equal amounts of PERV-B infected 293 cells and PERV-B infected 293 cells stably transfected with pSuper-pol2 were cultured. Three days later, the supernatant from these cells was collected, sterile filtrated and incubated with 5000 uninfected 293 cells in 96well plates for 4 days. Supernatants were removed and cells were lysed by three freeze/thaw cycles and three hour incubation with proteinase K in 1x PCR buffer at 56 °C. To inactivate proteinase K, the plate was incubated at 95 °C for 20 minutes. PCR was performed using cell lysate, *pol* specific primers (Pol-PK1/6, see Supplementary Table 2 online) and the AmpliTaq Gold Polymerase (Applied Biosystems). TCID₅₀ was calculated using the Spearmann-Karber method. Titration was performed in four replicates, 50µl virus-containing supernatant was diluted 1:2.

### Results

### Inhibition of PERV expression by synthetic siRNAs and selection of potent siRNA target sequences

Synthetic siRNAs targeting the genes *gag, pol* and env of PERV were designed according to published guidelines (Fig.1A) (Elbashir et al., 2002). The siRNAs gag1, gag2, pol2, pol4 and pol5 should be able to inhibit PERV-A, PERV-B and PERV-C due to conserved sequences. The siRNAs poll, pol3, env1, and env2 are specific for PERV-B. The siRNAs were transfected into PERV-B infected 293 cells and the inhibition of gene expression was measured by real-time reverse transcription (RT) PCR with different primer pairs flanking the siRNA target region (Fig. 1A). Some of the selected siRNAs failed to induce any silencing activity (e.g., gag1), whereas others suppressed viral gene expression to as little as 18% (Fig 1B). Based on these data the most effective inhibitory construct in terms of silencing PERV expression (siRNA Pol2) was selected.

### Inhibition of PERV expression after stable transfection of a shRNA expressing plasmid

In order to achieve permanent expression of this *pol*-specific siRNA, oligonucleotides corresponding to the pol2 region were ligated into the vector pSuper (Suppression of endogenous RNA) containing the polymerase III H1-RNA gene promoter (Brummelkamp, Bernards, and Agami, 2002). The plasmid was co-transfected into PERV-B infected 293 cells together with the plasmid pHygEGFP expressing the enhanced green fluorescent protein (EGFP) and providing hygromycin resistance.

Some of the isolated cell clones showed a strongly reduced expression of p27Gag protein, as shown by Western blot analysis. A strong reduction up to 90% was found in the cell clone A5. Although the target region is located within the *pol* gene, the degradation of the complete full-length mRNA coding for both Gag and Pol resulted in a concomitant decrease of Gag protein synthesis. Because the viral protease (also encoded by the *pol* gene) was silenced at the same time, the Gag protein was not processed, leading to a relative accumulation of the Gag precursor protein (60 kDa), whereas the other Gag proteins partially or completely disappeared (Fig. 2A). Virtually the same degree of inhibition could be demonstrated at the RNA level (Fig. 2B). This was shown by a quantitative real-time PCR using the primer pairs (pol2 up/down) flanking the shRNA binding site. However, when other oligonucleotide primers were employed located up- and downstream of the shRNA binding site, the level of viral RNA was not significantly higher, indicating a rapid decay of the full-length RNA molecule by the RISC complex.

### Inhibition of expression of full-length, but not spliced env RNA

Using a PCR method which allows to discriminate between full-length and spliced *env* RNA, it was shown, that the synthesis of the spliced *env* mRNA was not influenced by the expression of pol2 shRNA in clone A5 cells but the full-length mRNA was (Fig.2C). The viral *env* message is spliced in the nucleus and the viral precursor RNA cannot be processed by the RISC complex located in the cytoplasm (Zeng and Cullen, 2002). This result also shows that the spliced env RNA is expressed at similar levels in PERV-B infected 293 cells and A5 cells, indicating that the inhibition of expression of the full-length mRNA in A5 cells up to 14% is not due to a reduced promoter activity but due to the expression of pol2 shRNA.

After culturing these A5 cells for several months the gene silencing activity was still present and the plasmid pSuper was always detected by PCR in these cells (data not shown). The morphology and proliferation rate of these cells were not changed compared to the untransfected cells.

### Inhibition of PERV protein expression

To confirm results showing inhibition of PERV protein expression (Fig. 2A), an immunofluorescence assay was performed, measuring the expression of p27Gag in PERV-B infected cells, in cells from clone A5 and in uninfected 293 cells (Fig. 2D). Since the A5 cells were co-transfected with the plasmid pHygEGFP, these cells stably express EGFP together with the shRNA pol2. Clone A5 cells showed a strong reduction of p27Gag expression compared to the untreated PERV-B infected cells and at the same time EGFP is expressed (Fig. 2D).

### Inhibition of virus release

Finally, to study the release of infectious PERV by pSuper-pol2 transfected PERV-B infected cells, the titres of infectious particles in the supernatant of PERV-B infected cells and of clone A5 cells were determined. When equal volumes of the supernatants produced by an equal number of cells in the same time span were titrated on uninfected 293 cells, the TCID₅₀/ml in the supernatant of cells expressing the shRNAs against PERV *pol* was more than one log lower than that of untreated PERV-B infected 293 cells (TCID₅₀/ml_{PERV-B} = 190; TCID₅₀/ml_{A5} = 17) .

Using synthetic siRNAs corresponding to different parts of the viral genes *gag, pol* and *env*, several suitable target regions for RNAi within the PERV genome were identified, among them the pol2 sequence that proved to be the most effective in reducing virus expression. This is the first report showing suppression of a retrovirus other than HIV-1 and the first report showing suppression of an endogenous retrovirus. Since these experiments were performed in PERV infected 293 cells which produce viral particles at a relatively high level, the suppression of PERV by RNAi may well be more effective in pig cell lines or primary porcine cells producing less virus as shown previously (Denner et al., 2001).

The present invention also relates to the use of siRNA for the manufacture of a medicament for knock down the expression of target sequences in a pig.

The medicament of the invention is produced in a known way using the usual solid or liquid substrates or diluents and the common adjuvants used in pharmaceutical engineering and with an appropriate dosage depending on the intended mode of application. Preferred formulations are those forms suitable for oral administration, such as tablets, film tablets, lozenges, capsules, pills, powder, solutions, suspensions, or depot forms, for transdermal administration, such as solutions, suspensions, creams, emulsions, or band-aids, for intravenous infusion, or for subcutaneous injection, examples for both are solutions and suspensions. It will be understood that the specific dose level, frequency and period of administration of any particular mammal will depend upon a variety of factors including the activity of the specific compound employed, the age, body weight, general health, sex, diet time of administration, route of administration, rate of excretion, drug combination and the severity of the specific therapy.

The medicament can also contain, if desired, other PERV-attacking agents. The amount of active ingredient that is combined with the carrier materials to produce a single dosage form varies depending upon the host treated and the particular mode of administration. Furthermore, the siRNA of the present invention can be mixed with a pharmaceutically acceptable carrier or diluent in accordance with routine procedures.

In a further embodiment of the invention, said medicament is a vaccine or vaccine-adjuvant. In accordance with the present invention, the term vaccine composition relates to any composition which can be used as a vaccine. A vaccine means a therapeutic or prophylactic use of the medicament which attacks PERV. The forms or methods for manufacturing vaccine compositions according to the present invention are not particularly limited, and a composition in a desired form can be prepared by applying a single method available in the field of the art or methods in an appropriate combination. For the manufacture of a vaccine composition, aqueous media such as distilled water for injection and physiological saline, as well as one or more kinds of pharmaceutical additives available in the field of the art can be used. For example, buffering agents, pH adjusting agents, solubilizing aids, stabilizing agents, soothing agents, antiseptics, and the like can be used, and specific ingredients thereof are well known to those skilled in the art. The vaccine composition can also be prepared as a solid preparation such as a lyophilized preparation, and then prepared as an injection by adding a solubilizing agent such as distilled water for injection before use. Depending upon the manner of introduction, the compounds may be formulated in a variety of ways as discussed below. The concentration of therapeutically active compound in the formulation may vary from about 0.1 to 100 wt %. The vaccine composition may be administered alone or in combination with other treatments. In a preferred embodiment, the vaccine compositions are in a water-soluble form, such as pharmaceutically acceptable salts, which is meant to include both acid and base addition salts. The vaccine compositions can be prepared in various forms, such as injection solutions, suspensions and the like. The vaccine compositions may also include one or more of the following: carrier proteins, such as serum albumin; buffers; stabilizing agents; coloring agents and the like. Additives are well known in the art, and are used in a variety of formulations.

In addition, the siRNA of the invention can be typically used as vaccine adjuvant to enhance the protection afforded by animal or human vaccines that are considered weak (i.e. by providing diminished protection in terms of level, extent, and/or duration). The siRNA as vaccine-adjuvant will normally be administered separately from the vaccine, although it may be administered in combination with the vaccine as well. Administration of siRNA as vaccine-adjuvant can be subcutaneous, intravenous, parenteral, intramuscular, or in any other acceptable method. Preferably, the vaccine-adjuvant is administered prior to the administration of the vaccine and at the same site where the vaccine is to be administered. The formulations and pharmaceutical compositions contemplated by the above dosage forms can be prepared with conventional pharmaceutically acceptable excipients and additives, and by using conventional techniques. Other adjuvants may be administered either with the vaccine or together with the siRNA.

The transfer of the medicament in terms of siRNA may also be performed by means of a gene therapy. The most common method involves the introduction of genetic material into gene shuttles, for example retro viruses. The introduction and expression of siRNA in certain cell types can also be performed *in-vitro* and the modified cells are subsequently re-injected into the organism. The recombination of the vehicle genome or the cell genome, respectively, with the nucleic acid sequence encoding the siRNA follows standard techniques, whereas targeting and the stable incorporation of the new gene represent current research topics.

The RNAi strategy used here could lead to the generation of transgenic pigs providing safe xenotransplants and is therefore of considerable medical importance. Transgenic mice have recently been described (Carmell et al., 2003) in which expression of shRNA lead to the silencing of the Neil1 gene, a DNA N-glycosylase, in all organs tested demonstrating the feasibility of this concept. In order to generate transgenic pigs expressing shRNA-pol2, a lentiviral vector may be useful. As has been shown previously, pig strains and single individuals from the same strain differ in their ability to release PERV from mitogen stimulated normal blood cells (Tacke, Specke, and Denner, 2003). Conventional breeding and selection of such low-virus producer pigs together with the creation of transgenic animals may result in a highly reduced PERV expression in cells and organs suitable for safe xenotransplantations. The siRNA corresponding to a highly conserved sequence of the *pol* gene of all PERVs will also suppress expression of defective proviruses, thus preventing complementation. In order to reduce the virus release to zero, additional shRNA targeting PERV at different conserved regions will be useful. Since the RNAi mechanism seems to be sequence specific, gene silencing of vital cellular proteins should not occur in transgenic pigs, although this has still to be demonstrated experimentally as some RNAi off-target effects have been recently reported (ref. 26 and own unpublished data). In addition to the work on other strategies to prevent PERV transmission (Fiebig et al., 2003; Oldmixon et al., 2002; Tacke, Specke, and Denner, 2003), extensive virological characterisation of PERVs adapted to human cells (Denner et al., 2003), investigation of the *in vitro* and *in vivo* host range (Denner et al., 2001; Loss et al., 2001; Patience, Takeuchi, and Weiss, 1997; Specke, Rubant, and Denner, 2001; Specke et al., 2000), as well as sensitive and specific detection methods were developed to facilitate monitoring of PERV transmission in future clinical xenotransplantations (Tacke et al., 2001). Together these investigations allow a reliable evaluation of the potential risk of PERVs in xenotransplantation.

### Legends

Fig.1. Localisation and efficacy of siRNAs targeting PERV. (A), Schematic presentation of the PERV genome with the localisation of siRNA target sequences and oligonucleotide primers used for detection of viral RNA in PCR assays. SD and SA indicate splice donor and acceptor sites. (B), Expression of viral RNA as measured by quantitative real time RT-PCR after transfection of synthetic siRNAs into PERV-B infected human 293 kidney cells, expressed as percentage of mRNA synthesis in PERV-B infected 293 cells transfected with an irrelevant control siRNA.
Fig. 2. Inhibition of PERV p27Gag expression after stable transfection of pSuper-pol2 shRNA. (A), Western blot assay measuring viral Gag protein in untreated 293 cells, in PERV-B infected 293 cells and in PERV-B infected 293 cells stably transfected with pSuper-pol2 (cell clone A5). Gag protein expression was significantly inhibited by the siRNA and due to the inhibition of the viral protease a relative accumulation of the precursor Gag protein was observed. (B), Viral RNA was processed as measured by real time RT-PCR with different primer pairs (gag1 up/down in violet, pol2 up/down in green, pol3-4-5 up/down in blue). PERV expression was calculated as the percentage of mRNA expression in PERV-B infected 293 cells. (C), The expression rate of spliced and unspliced viral RNA was quantified in PERV-B infected 293 cells and clone A5 cells expressing pSuper-pol2 shRNAs using primers flanking the splice donor and splice acceptor site. The results demonstrate the effective inhibition of full-length but not spliced env RNA. (D), Immunofluorescence assays also demonstrate significant inhibition of p27Gag expression in PERV-B infected 293 cells transfected with pSuper-pol2 in comparison to untreated PERV-B infected 293 cells. Cells were treated with an antiserum against p27Gag and a TRITC-labelled secondary antibody. The expression of EGFP indicates stable co-transfection of pSuper-pol2 and pHygEGFP.

While the invention has been described in terms of preferred embodiments, the skilled artisan will appreciate that various modifications, substitutions, omissions and changes may be made without departing from the spirit thereof. Accordingly, it is intended that the scope of the present invention be limited solely by the scope of the following claims, including equivalents thereof. Therefore, as will be apparent to those skilled in the art in which the invention is addressed, the present invention may be embodied in forms other than those specifically disclosed above without departing from the spirit or essential characteristics of the invention. The particular embodiments of the present invention, described above, are therefore to be considered in all respects as illustrative and not restrictive. The scope of the present invention is as set forth in the appended claims rather than being limited to the examples contained in the foregoing description.

### References

Billy, E., Brondani, V., Zhang, H., Muller, U., and Filipowicz, W. (2001). Specific interference with gene expression induced by long, double-stranded RNA in mouse embryonal teratocarcinoma cell lines. *Proc Natl Acad Sci U S A* **98**(25), 14428-33.
Brummelkamp, T. R., Bernards, R., and Agami, R. (2002). A system for stable expression of short interfering RNAs in mammalian cells. *Science* **296**(5567), 550-3.
Caplen, N. J., Parrish, S., Imani, F., Fire, A., and Morgan, R. A. (2001). Specific inhibition of gene expression by small double-stranded RNAs in invertebrate and vertebrate systems. *Proc Natl Acad Sci U S A* **98**(17), 9742-7.
Carmell, M. A., Zhang, L., Conklin, D. S., Hannon, G. J., and Rosenquist, T. A. (2003). Germline transmission of RNAi in mice. *Nat Struct Biol* **10**(2), 91-2.
Cooper, D. K. (2003). Clinical xenotransplantion--how close are we? Lancet **362**(9383), 557-9.
Denner, J. (1998). Immunosuppression by retroviruses: implications for xenotransplantation. *Ann N Y Acad Sci.* **862**, 75-86.
Denner, J., Specke, V., Schwendemann, J., and Tacke, S. J. (2001). Porcine endogenous retroviruses (PERVs): adaptation to human cells and attempts to infect small animals and non-human primates. *Ann Transplant* **6**(3), 25-33.
Denner, J., Specke, V., Thiesen, U., Karlas, A., and Kurth, R. (2003). Genetic alterations of the long terminal repeat of an ecotropic porcine endogenous retrovirus during passage in human cells. V*irology* **314**(1), 125-33.
Elbashir, S. M., Harborth, J., Weber, K., and Tuschl, T. (2002). Analysis of gene function in somatic mammalian cells using small interfering RNAs. *Methods* **26**(2), 199-213.
Elbashir, S. M., Lendeckel, W., and Tuschl, T. (2001). RNA interference is mediated by 21- and 22-nucleotide RNAs. *Genes Dev* **15**(2), 188-200.
Fiebig, U., Stephan, O., Kurth, R., and Denner, J. (2003). Neutralizing antibodies against conserved domains of p15E of porcine endogenous retroviruses: basis for a vaccine for xenotransplantation? *Virology* **307**(2), 406-13.
Hammond, S. M., Bernstein, E., Beach, D., and Hannon, G. J. (2000). An RNA-directed nuclease mediates post-transcriptional gene silencing in Drosophila cells. *Nature* **404**(6775), 293-6.
Irgang, M., Sauer, I. M., Karlas, A., Zeilinger, K., Gerlach, J. C., Kurth, R., Neuhaus, P., and Denner, J. (2003). Porcine endogenous retroviruses: no infection in patients treated with a bioreactor based on porcine liver cells. *J Clin Virol* **28**(2), 141-54.
Livak, K. J., and Schmittgen, T. D. (2001). Analysis of relative gene expression data using real-time quantitative PCR and the 2(-Delta Delta C(T)) Method. *Methods* **25**(4), 402-8.
Loss, M., Arends, H., Winkler, M., Przemeck, M., Steinhoff, G., Rensing, S., Kaup, F., Hedrich, H., Winkler, M., and Martin, U. (2001). Analysis of potential porcine endogenous retrovirus (PERV) transmission in a whole-organ xenotransplantation model without interfering microchimerism. *Transpl Int* **14**(1), 31-37.
McManus, M. T., Haines, B. B., Dillon, C. P., Whitehurst, C. E., van Parijs, L., Chen, J., and Sharp, P. A. (2002). Small interfering RNA-mediated gene silencing in T lymphocytes. *J Immunol* **169** (10), 5754-60.
Oldmixon, B. A., Wood, J. C., Ericsson, T. A., Wilson, C. A., White-Scharf, M. E., Andersson, G., Greenstein, J. L., Schuurman, H. J., and Patience, C. (2002). Porcine endogenous retrovirus transmission characteristics of an inbred herd of miniature swine. *J Virol* **76**(6), 3045-8.
Paddison, P. J., Caudy, A. A., Bernstein, E., Hannon, G. J., and Conklin, D. S. (2002). Short hairpin RNAs (shRNAs) induce sequence-specific silencing in mammalian cells. *Genes Dev* **16**(8), 948-58.
Paradis, K., Langford, G., Long, Z., Heneine, W., Sandstrom, P., Switzer, W., Chapman, L., Lockey, C., Onions, D., and Otto, E. (1999). Search for cross-species transmission of porcine endogenous retrovirus in patients treated with living pig tissue. The XEN 111 Study Group. *Science* **285**(5431), 1236-1241.
Patience, C., Switzer, W., Takeuchi, Y., Griffiths, D., Goward, M., Heneine, W., Stoye, J., and Weiss, R. (2001). Multiple groups of novel retroviral genomes in pigs and related species. *J Virol* **75**(6), 2771-2775.
Patience, C., Takeuchi, Y., and Weiss, R. (1997). Infection of human cells by an endogenous retrovirus of pigs. *Nat Med* **3**(3), 282-286.
Paul, C. P., Good, P. D., Winer, I., and Engelke, D. R. (2002). Effective expression of small interfering RNA in human cells. *Nat Biotechnol* **20**(5), 505-8.
Specke, V., Rubant, S., and Denner, J. (2001). Productive infection of human primary cells and cell lines with porcine endogenous retroviruses. *Virology* **285**(2), 177-180.
Specke, V., Tacke, S., Boller, K., Schwendemann, J., and Denner, J. (2000). Porcine endogenous retroviruses: in vitro host range and attempts to establish small animal models. *J Gen Virol.* **82(Pt4)**, 837-844.
Stein, P., Svoboda, P., Anger, M., and Schultz, R. M. (2003). RNAi: mammalian oocytes do it without RNA-dependent RNA polymerase. *Rna* **9**(2), 187-92.
Sui, G., Soohoo, C., Affar el, B., Gay, F., Shi, Y., and Forrester, W. C. (2002). A DNA vector-based RNAi technology to suppress gene expression in mammalian cells. *Proc Natl Acad Sci U S A* **99**(8), 5515-20.
Tacke, S., Bodusch, K., Berg, A., and Denner, J. (2001). Sensitive and specific immunological detection methods for porcine endogenous retroviruses applicable to experimental and clinical xenotransplantation. *Xenotransplantation* **8**(2), 125-135.
Tacke, S., Specke, V., and Denner, J. (2003). Differences in release and determination of subtype of porcine endogenous retroviruses (PERV) produced by stimulated normal pig blood cells. *Intervirology* **46**, 17-24.
Yang, D., Lu, H., and Erickson, J. W. (2000). Evidence that processed small dsRNAs may mediate sequence-specific mRNA degradation during RNAi in Drosophila embryos. *Curr Biol* **10**(19), 1191-200.
Zeng, Y., and Cullen, B. R. (2002). RNA interference in human cells is restricted to the cytoplasm. *Rna* **8**(7), 855-60.

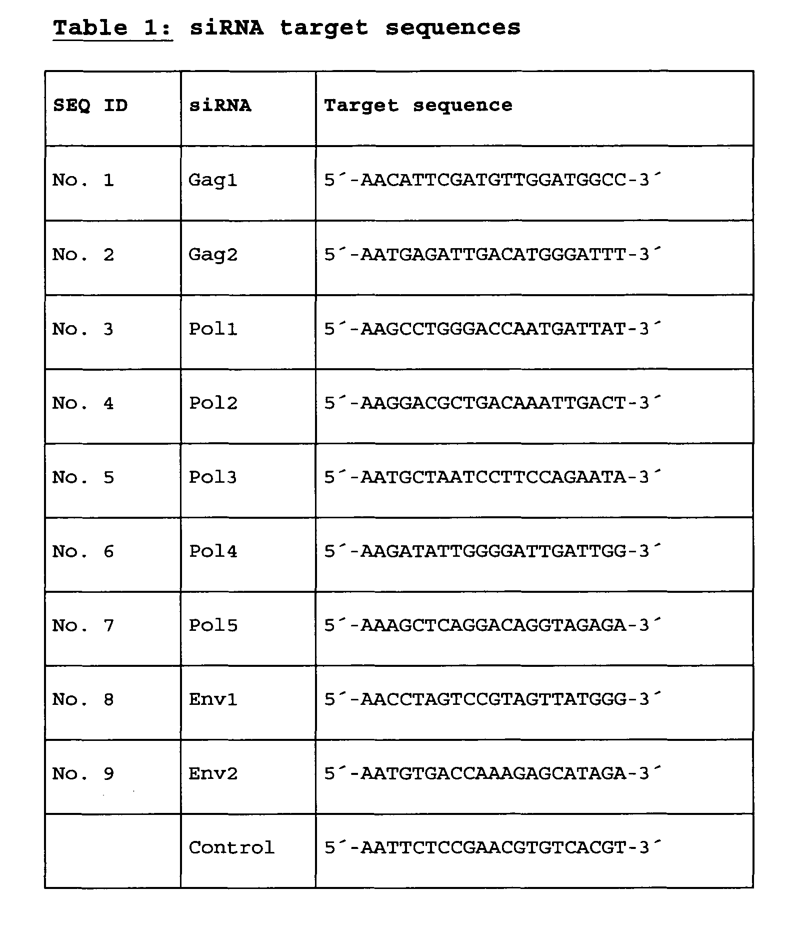

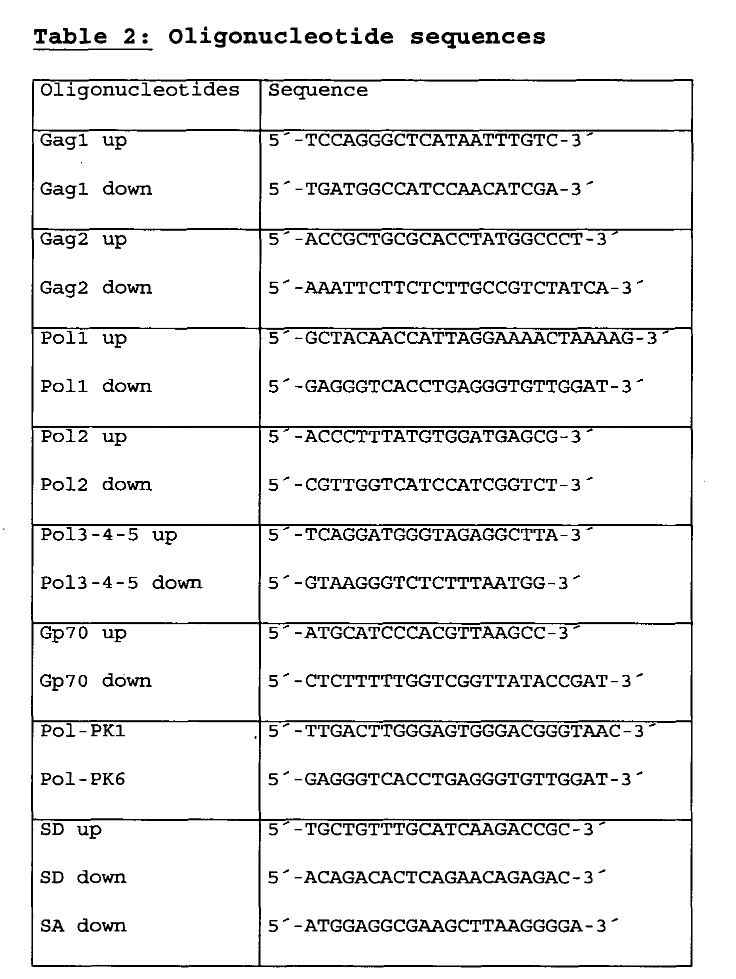

All oligonucleotides/siRNAs were selected according to the published sequence of PERV subtype B (Gen-Bank AJ133816)

## Claims

1. siRNA for knock down the expression of porcine endogenous retrovirus (PERVs),
**characterized in that**,
a target sequence of the siRNA is selected from the group comprising a sequence according to SEQ ID No. 1 - SEQ ID No. 9.

2. Vector comprising the siRNA according to claim 1.

3. Cell comprising the vector according to claim 2.

4. Organism comprising the cell according to claim 3.

5. Organism according to claim 4,
**characterized in that**,
the organism is a pig.

6. Use of siRNA for the manufacture of a medicament for knock down the expression of target sequences in a pig.

7. Use according to claim 6,
**characterized in that**,
the siRNA is the siRNA according to claim 1.
